Europäisches Patentamt

European Patent Office

Office européen des brevets

(19)

(11) Numéro de publication : **0 465 337 A1**

(12) # DEMANDE DE BREVET EUROPEEN

(21) Numéro de dépôt : **91401798.3**

(22) Date de dépôt : **28.06.91**

(51) Int. Cl.⁵ : **A61K 9/16**, A61K 9/50,
A61K 9/52, A61K 9/54,
A23K 1/00

(30) Priorité : **29.06.90 FR 9008279**

(43) Date de publication de la demande :
**08.01.92 Bulletin 92/02**

(84) Etats contractants désignés :
**AT BE CH DE DK ES FR GB GR IT LI LU NL SE**

(71) Demandeur : **RHONE-POULENC NUTRITION
ANIMALE
Rue Marcel Lingot
F-03600 Commentry (FR)**

(72) Inventeur : **Autant, Pierre
14 rue de Pourcheroux
F-03600 Commentry (FR)**
Inventeur : **Cartillier, André
Champfromenteau
F-03600 Commentry (FR)**

(74) Mandataire : **Le Pennec, Magali et al
RHONE-POULENC SANTE, Service Brevets,
20 Avenue Raymond Aron
F-92165 Antony Cédex (FR)**

(54) **Procédé de lissage de granulés de principes actifs.**

(57)    La présente invention concerne un procédé de lissage de principes actifs alimentaires et/ou médicamenteux destinés à l'alimentation ou au traitement des ruminants par pulvérisation sur lesdits principes actifs d'une solution d'un ou plusieurs principes actifs de gommes et/ou de sucres.
   Lesdits principes actifs lissés sont ensuite enrobés par un polymère permettant leur protection lors du passage dans le rumen.

EP 0 465 337 A1

La présente invention concerne un procédé de lissage de granulés de principes actifs. Elle concerne plus particulièrement un procédé de lissage de granulés de principes actifs destinés à l'alimentation et/ou au traitement des ruminants.

Il est connu par exemple selon les brevets US 4 181 708, 4 181 709 et 4 181 710 de préparer des granulés adaptés à l'administration chez les ruminants composés d'un coeur de substance active et d'un enrobage à base d'une substance hydrophobe et d'un polymère résistant au pH neutre du rumen et dégradable au pH plus acide de la caillette.

Le coeur de ces granulés, composé du principe actif se présente sous forme granulaire. Il est obtenu par mélange à sec des matières actives, des liants et éventuellement des agents de neutralisation et des agents de densification, mélange qui est ensuite mouillé, transformé en une masse pâteuse qui par extrusion à la température ambiante et sphéronisation est granulée. Les granulés humides sont séchés dans des fours ou des lits fluidisés.

Les granulés obtenus sont directement utilisés pour l'opération d'enrobage par le mélange de polymère, de substance hydrophobe et de charges. Ce mélange d'enrobage est mis en solution dans un solvant organique puis pulvérisé sur les granulés.

Pour obtenir une protection correcte du principe actif, il est nécessaire de pulvériser au moins 20 g du mélange polymère et substance hydrophobe pour 100 g de matière active granulée et la couche d'enrobage a une épaisseur d'environ 150 µm (voir exemple 1 du brevet US 4 181 710).

Le polymère étant un composé onéreux, l'industrie cherche depuis longtemps un moyen de diminuer son pourcentage pondéral dans le granulé final, tout en conservant une bonne protection de la matière active lors de son passage dans le rumen.

Cet objectif a été atteint en utilisant comme coeur de principe actif devant subir l'enrobage, un granulé ayant subi une opération supplémentaire de lissage. On entend par opération de "lissage" une opération qui permet de diminuer la surface spécifique du granulé par diminution des irrégularités de surface. Ce granulé "lissé", pour une même quantité de matière active, exigera alors une quantité d'enrobant inférieure, la quantité d'enrobant nécessaire à une bonne protection étant liée à la surface spécifique du granulé de départ.

L'étape de lissage est réalisée selon la présente invention par dépôt sur le granulé de principe actif d'une solution de principes actifs et/ou de sucres et/ou de gommes. On préfère utiliser une solution aqueuse de principe actif et tout particulièrement une solution de chlorhydrate de lysine.

Les granulés de base devant subir l'opération de lissage peut être choisi parmi les cristaux de chlorhydrate de lysine ou encore ils peuvent être préparés selon le mode opératoire des brevets US 4 181 708 ou US 4 181 710 par mélange d'un ou plusieurs principes sous forme de poudre, d'un agent liant et éventuellement d'un diluant et/ou d'une charge.

Les principes actifs sont choisis notamment parmi les amino acides tels que la méthionine, la lysine ou ses sels, la phénylalanine, l'histidine, l'arginine, la tyrosine, les médicaments tels que les vitamines, les antibiotiques, les antiparasitaires, ... les protéines. On préfère parmi les principes actifs utiliser la lysine. Ainsi on obtient selon cette préférence un granulé homogène constitué d'un coeur de lysine et lissé par une pellicule de lysine. Les agents diluants et les charges sont choisis parmi les sucres, la cellulose, la silice. Les agents liants sont choisis parmi les dérivés de la cellulose tels que l'hydroxypropylméthylcellulose, les gommes, ainsi que tous les dérivés connus dans l'industrie alimentaire pour épaissir ou favoriser la préparation des comprimés.

Les différents constituants sont granulés par toute technique connue de l'homme de l'art, par exemple après mouillage par extrusion à travers une filière, puis séchage, ils peuvent aussi subir un compactage puis un broyage.

Les granulés obtenus sont tamisés de façon à conserver une répartition de granulés comprise entre 200 et 4000 µm et de préférence entre 500 µm et 2500 µm.

Ces granulés présentent une surface irrégulière et exigent pour leur protection vis-à-vis de leur passage trans-rumen une quantité d'enrobant importante. Afin de diminuer l'épaisseur de la couche d'enrobage nécessaire, on a diminué, par le procédé de la présente invention, l'irrégularité de surface des granulés par un traitement de lissage à l'aide d'une solution d'un des principes actifs et/ou de sucre et/ou de gomme.

On préfère utiliser, pour l'opération de lissage, une solution de chlorhydrate de lysine la plus concentrée possible. Ainsi, une concentration de chlorhydrate de lysine de 40 à 150 g pour 100 g d'eau ce qui représente une solution contenant entre 28 et 60 % en poids de lysine est notamment utilisée. Il est bien évident que toute solution plus diluée est utilisable dans le cadre de la présente invention. Mais toute dilution exigera une étape plus longue de pulvérisation. Aussi, pour une meilleure économie du procédé, il est préférable d'utiliser une solution de "lissage" aussi concentrée que possible, la solubilité de la lysine augmentant nettement avec la température, pour des concentrations supérieure à environ 50 % (c'est à dire contenant au moins 100 g de chlorhydrate de lysine pour 100 g d'eau) il est nécessaire de maintenir la solution à une température comprise entre 50 et 70°C.

La couche de lissage peut varier en poids dans de larges proportions, surtout lorsqu'elle est constituée d'une matière active. Ainsi, une couche représentant en poids entre 10 et 150 % du poids du granulé de départ est utilisable mais une quantité comprise entre 50 et 110 % est préférée et une quantité comprise entre 70 et 100 est plus intéressante lorsque le coeur est constitué de cristaux de chlorhydrate de lysine.

L'épaisseur de la couche de lissage varie avec le diamètre du granulé de départ pour un même pourcentage en poids d'agent de lissage, elle est liée au diamètre, à la répartition granulométrique et à la forme des granulés. On préfère utiliser une couche de lissage comprise entre 20 et 200 µm.

La couche d'enrobage, protectrice vis à vis du rumen, est ensuite pulvérisée selon la technique décrite dans les brevet américains précités.

L'enrobage contient au moins un élément qui est choisi parmi les polymères, les copolymères ou les mélanges basiques dont le taux d'azote est compris entre 2 et 14 % et le poids moléculaire est compris entre 50 000 et 500 000. Pour la définition des polymères et copolymères on se reportera à leur définition colonne 7 du brevet US 4 181 710 qui est inclus dans la présente demande par référence. On préfère parmi les copolymères utiliser le copolymère styrène vinyl-2 pyridine (contenant 50 à 80 % en poids de vinyl-2 pyridine et 20 % à 50 % de styrène).

L'enrobant contient aussi une substance hydrophobe choisie de préférence parmi les acides gras ayant 12 à 32 atomes de carbone. Les substances hydrophobes sont aussi décrites dans le brevet US 4 181 710 qui est également inclus dans la présente demande par référence. On préfère parmi les substances hydrophobes utiliser l'acide stéarique.

L'enrobant préféré selon l'invention a la composition pondérale suivante :

10-30 % copolymère vinyl-2 pyridine - styrène

70-90 % acide stéarique.

Le mélange enrobant contenant le copolymère et la substance hydrophobe est mis en solution dans un solvant halogéné, un alcool, un éther, une cétone ou un mélange de ces solvants. Il est tout particulièrement avantageux d'utiliser un mélange éthanol/dichloro-1,2 éthane, éthanol/chlorure de méthylène, éthanol/acétone. La solution de mélange enrobant est pulvérisée sur les granulés "lissés" à l'aide d'un lit fluidisé ou de tout autre appareil de pulvérisation. On préfère utiliser un appareil de pulvérisation connu sous la dénomination de spray-coating, type UNIGLATT équipé d'une cuve WURSTER.

La quantité d'enrobant utilisée, exprimée en matière sèche par rapport au granulé "lissé" est comprise notamment entre 10 et 30 % et de préférence entre 15 et 25 % ce qui pour des granulés ayant un diamètre moyen compris entre 500 µm et 1400 µm représente une épaisseur d'enrobage comprise entre 20 et 70 µm.

Les granulés obtenus après enrobage présentent une stabilité vis-à-vis du PH neutre du rumen bien améliorée par rapport aux granulés de l'art antérieur n'ayant pas subi d'opération de lissage.

La présente invention sera plus complètement décrite à l'aide des exemples suivants qui ne doivent pas être considérés comme limitatifs de l'invention.


EXEMPLE 1 GRANULATION

Préparation de granulé de chlorhydrate de lysine par extrusion et sphéronisation


**Matériel mis en oeuvre :**
Mélangeur de capacité 50 L ( système planétaire )
Extrudeuse PHARMEX 45
Sphéroniseur SPHAEROMAT 400
Séchoir en lit fluidisé.
**Matières premières :**
Chlorhydrate de lysine broyée au broyeur FORPLEX diamètre 400 µm
Hydroxy propyl méthyl cellulose
Cellulose microcristalline AVICEL
**Mode opératoire :**
a) Empâtage
Dans le mélangeur, on introduit

```
        Chlorhydrate de lysine de diamètre 400 µm         2550 g

        cellulose microcristalline                         450 g
```

On homogénéise à sec pendant 1 minute.

Puis on introduit une solution aqueuse de liant préparée à l'avance et formée de :

```
        eau                             1227,9 g
        hydroxypropylméthylcellulose      92,1 g
```

On agite pendant 1 minute 30 secondes après la fin de l'introduction.

b) Extrusion

La pâte obtenue est extrudée à l'aide de l'extrudeuse PHARMEX 45 équipée d'une filière de diamètre 0,8 mm - vitesse de rotation de la vis 90 tr/mn.

c) Sphéronisation

La totalité de l'extrudat est immédiatement introduite dans le sphéroniseur en rotation - vitesse 1000 tr/mn - durée du traitement : 2 mn.

d) Séchage en lit fluidisé

Température de l'air : 60°C.

e) granulométrie des sphérules obtenues

```
             diamètre ≤  0,5  mm        13,8 %
      0,5 <  diamètre ≤  0,63 mm         8,4 %
      0,63 < diamètre ≤  0,8  mm        16,2 %
      0,8 <  diamètre ≤  1    mm        28,1 %
      1 <    diamètre ≤  1,25 mm        19,3 %
      1,25 < diamètre ≤  1,25 mm        14,2 %*
```

* Cette fraction de diamètre supérieur à 1,25 mm inclut des agglomérats issus du séchage.


EXEMPLE 2 LISSAGE

Mode opératoire du lissage

400 g des particules à traiter (granulés de l'exemple 1) de diamètre compris entre 0,63 et 0,80 mm sont introduits dans l'appareil UNIGLATT.

La fluidisation est mise en route et l'on pulvérise la solution de lissage testée.

Après lissage, on recueille le produit et on réalise l'enrobage proprement dit sur 350 g de particules lissées, de la façon décrite ci-dessous.

Préparation des solutions de lissage :

La quantité désirée de monochlorhydrate de lysine (titre 99,9 %) est dissoute à environ 30°C dans l'eau distillée.

La teneur en chlorhydrate de lysine est limitée par sa solubilité dans l'eau.

Réglages de l'appareil :

Toutes les opérations de lissage ont été réalisées dans les conditions suivantes :

|  |  |
|---|---|
| - charge granulés | 400 g |
| - débit air de fluidisation | 100 m$^3$/h |
| - température d'entrée de l'air | 40°C |
| - pression de pulvérisation | 1,5 bar |
| - débit de pulvérisation de la solution de lissage | 14 ml/mn |
| - température de la solution | 30°C |

EXEMPLE 3 ENROBAGE

Matériel d'enrobage :

Appareil de spray-coating GLATT type UNIGLATT cuve WURSTER 5 pouces.

Mode opératoire d'enrobage :

Solution enrobante :
Composition pour 100 g de solution:

|  |  |
|---|---|
| - copolymère vinyl-2 pyridine-styrène (70-30) | 1,93 g |
| - acide stéarique pur | 7,74 g |
| - éthanol | 35,06 g |
| - dichloro-1-2 éthane | 55,26 g |

Réglage du lit fluidisé :

|  |  |
|---|---|
| - Charge de particules de diamètre 0,63-0,80 mm | 350 g |
| - Débit air de fluidisation | 100 Nm$^3$/h |
| - Température d'entrée de l'air | 30°C |
| - Débit de pulvérisation de la solution enrobante | 11 ml/mn |
| - Pression de l'air de pulvérisation | 1,5 bar |
| - Température de la solution enrobante | 40°C |

Mode opératoire :

Les particules à enrober sont chargées dans la cuve de l'UNIGLATT. La fluidisation est mise en route et l'on commence la pulvérisation de la solution enrobante. On effectue des prélèvements de environ 10 g de produit après pulvérisation de :
. 362 g de solution correspondant à 35 g d'enrobant sec soit 10 % de taux d'enrobage
. 724 g de solution correspondant à 70 g d'enrobant sec soit 20 % de taux d'enrobage
. 1086 g de solution correspondant à 105 g d'enrobant sec soit 30 % de taux d'enrobage
Le taux d'enrobage étant défini comme le poids d'enrobant sec pulvérisé sur 100 g de particules initiales.
La taux de relargage en lysine HCl est déterminé sur chaque prélèvement (voir tableau 1).

EXEMPLE 4

Un essai comparable aux essais 1, 2 et 3 a été réalisé avec du chlorhydrate de lysine présentant un diamètre après granulation de :

– 0,5 à 0,63 mm
– 0,63 à 0,80 mm
– 0,80 à 1,0 mm
– 1,0 à 1,4 mm

pour un taux de lissage de 40 g de chlorhydrate de lysine pour 100 g de granulés. Les résultats reportés sur le tableau 2 prouvent que la protection est nettement plus facile à taux d'enrobage équivalent et faible (10 %) lorsque le granulé présente un diamètre plus élevé.

EXEMPLE 5

On reproduit l'exemple 4 avec un taux de lissage de 10 % et différentes épaisseurs d'enrobant. Les résultats sont exprimés dans le tableau 3.

EXEMPLE 6

On reproduit les exemples 2 et 3 en remplaçant le granulé à base de chlorhydrate de lysine, de cellulose microcristalline et d'hydroxypropylméthylcellulose par des particules de chlorhydrate de lysine simplement tamisées entre 0,63 et 0,80 mm. Le lissage et l'enrobage sont effectués avec les solutions décrites dans ces exemples.

Le tableau 4 indique de la même manière que le tableau 1 les résultats des taux de relargage en chlorhydrate de lysine.

## TABLEAU 1 : LISSAGE DE GRANULES PAR SOLUTION DE LYS./HCl

### INFLUENCE SUR LA QUALITE DE L'ENROBAGE

| N° ESSAI | LISSAGE eau ml/ 100 g coeur | LISSAGE Lys g/ 100 g coeur | diam. ap. lis µm | TAUX ENROBAGE g% g coeur | EPAISSEUR ENROBAGE µm | RELARG. pH6/24 h % | RELARG. pH6/5 h % |
|---|---|---|---|---|---|---|---|
| C1 | 0 | 0 | 715 | 10 | 14,4 | 88,8 | 49,1 |
|  |  |  |  | 20 | 27,8 | 25,4 | 7 |
|  |  |  |  | 30 | 40,4 | 8,5 | 0 |
| C2 | 50 | 0 | 715 | 10 | 14,4 | 100 | 88,6 |
|  |  |  |  | 20 | 27,8 | 45,9 | 12 |
|  |  |  |  | 30 | 40,4 | 13,5 | 0 |
| 1 | 50 | 10 | 738 | 10 | 14,9 | 31,2 | 11,3 |
|  |  |  |  | 20 | 28,9 | 3,5 | 0 |
|  |  |  |  | 30 | 41,7 | 0 | 0 |
| 2 | 50 | 20 | 760 | 10 | 15,4 | 18,4 | 5,4 |
|  |  |  |  | 20 | 29,6 | 2,6 | 0 |
|  |  |  |  | 30 | 42,9 | 2,5 | 1,2 |
| 3 | 25 | 10 | 738 | 10 | 14,9 | 50,2 | 30,3 |
|  |  |  |  | 20 | 28,9 | 10,8 | 4,3 |
|  |  |  |  | 30 | 41,7 | 4,5 | 0 |
| 4 | 12,5 | 10 | 738 | 10 | 14,9 | 55,5 | 28,6 |
|  |  |  |  | 20 | 28,9 | 11,1 | 3,3 |
|  |  |  |  | 30 | 41,7 | 6,2 | 0 |

EP 0 465 337 A1

## TABLEAU 2 : INFLUENCE DE LA GRANULOMETRIE INITIALE

Composition de la solution de lissage :

|  | | |
|---|---|---|
| eau | 100 | g%g particules |
| lysine HCl | 40 | initiales |

| N° ESSAI | GRANULOMETRIE INITIALE mm | diam. moyen ap. lissage mm | TAUX ENROBAGE * | EPAISSEUR ENROBAGE μm | RELARG. pH6/24 h |
|---|---|---|---|---|---|
| 1 | 0,5 - 0,63 | 0,63 | 10 | 12 | 84,9 |
|   |            |      | 20 | 23 | 6 |
|   |            |      | 30 | 33 | 4,3 |
| 2 | 0,63 - 0,8 | 0,8 | 10 | 15 | 40 |
|   |            |     | 20 | 29 | 0 |
|   |            |     | 30 | 42 | 0,8 |
| 3 | 0,8 - 1,0 | 1 | 10 | 19 | 44,4 |
|   |           |   | 20 | 36 | 4,6 |
|   |           |   | 30 | 52 | 2,3 |
| 4 | 1,0 - 1,4 | 1,34 | 10 | 25 | 21,7 |
|   |           |      | 20 | 48 | 6,3 |
|   |           |      | 30 | 70 | 6,7 |

*   g enrobant % g particules lissées

EP 0 465 337 A1

## TABLEAU 3 : INFLUENCE DE LA GRANULOMETRIE INITIALE

Lissage "faible"

Composition de la solution de lissage :

| eau | 50 | g%g particules |
|-----|-----|-----|
| lysine HCl | 10 | initiales |

| N° ESSAI | GRANULOMETRIE INITIALE mm | diam. moyen ap. lissage mm | TAUX ENROBAGE * | EPAISSEUR ENROBAGE μm | RELARG. pH6/24 h |
|---|---|---|---|---|---|
| 1 | 0,5 - 0,63 | 0,58 | 10 | 11 | 100 |
|   |             |      | 20 | 21 | 11,2 |
|   |             |      | 30 | 30 | 3,6 |
| 2 | 0,63 - 0,8 | 0,74 | 10 | 14 | 97,8 |
|   |             |      | 20 | 27 | 7,2 |
|   |             |      | 30 | 39 | 2,4 |
| 3 | 0,8 - 1,0 | 0,98 | 10 | 18 | 71,8 |
|   |             |      | 20 | 35 | 7,6 |
|   |             |      | 30 | 51 | 3,5 |
| 4 | 1,0 - 1,4 | 1,24 | 10 | 23 | 63,3 |
|   |             |      | 20 | 45 | 13,3 |
|   |             |      | 30 | 65 | 9,8 |

\*    g enrobant % g particules lissées

EP 0 465 337 A1

EP 0 465 337 A1

TABLEAU 4 : LISSAGE DE PARTICULES DE LYSINE PAR SOLUTION DE LYS./HCl
INFLUENCE SUR LA QUALITE DE L'ENROBAGE

| N° ESSAI | LISSAGE eau ml/ 100 g coeur | LISSAGE Lys g/ 100 g coeur | diam. ap. lis µm | TAUX ENROBAGE g% g coeur | EPAISSEUR ENROBAGE µm | RELARG. pH6/24 h % | RELARG. pH6/5 h % |
|---|---|---|---|---|---|---|---|
| C1 | 50 | 0 | 715 | 10 | 14,4 | 100 | 94,9 |
| | | | | 20 | 27,9 | 37,8 | 16,9 |
| | | | | 30 | 40,2 | 19,1 | 7,5 |
| 1 | 25 | 10 | 738 | 10 | 14,9 | 98,8 | 43,9 |
| | | | | 20 | 28,8 | 19,7 | 8 |
| | | | | 30 | 41,7 | 12,1 | 4,7 |
| 2 | 25 | 20 | 760 | 10 | 15,4 | 100 | 33,8 |
| | | | | 20 | 29,6 | 22,6 | 5,8 |
| | | | | 30 | 42,9 | 9,1 | 2,3 |
| 3 | 12,5 | 10 | 738 | 10 | 14,9 | 96 | 44,6 |
| | | | | 20 | 28,9 | 28,1 | 10,9 |
| | | | | 30 | 41,7 | 16,1 | 4,9 |
| 4 | 50 | 20 | 760 | 10 | 15,4 | 100 | 57,6 |
| | | | | 20 | 29,6 | 29,6 | 12,8 |
| | | | | 30 | 42,9 | 21,8 | 5 |
| 5 | 31,2 | 25 | 770 | 10 | 15,6 | 78,9 | 18,9 |
| | | | | 20 | 30 | 22,9 | 6,1 |
| | | | | 30 | 43,5 | 9,1 | 2,1 |
| 6 | 62,5 | 50 | 818,5 | 10 | 16,55 | 35,8 | 10,8 |
| | | | | 20 | 31,9 | 13,9 | 2,3 |
| | | | | 30 | 46,3 | 8,7 | 1,7 |
| 7 | 93,7 | 75 | 861,6 | 10 | 17,4 | 21,5 | 6 |
| | | | | 20 | 33,6 | 2,7 | 0 |
| | | | | 30 | 48,7 | 0 | 0 |

**Revendications**

**1** - Procédé de "lissage" de granulés de principes actifs alimentaires et/ou médicamenteux destinés à l'alimentation ou au traitement des ruminants caractérisé en ce que l'on pulvérise sur les dits granulés une solution d'un ou plusieurs principes actifs, de gommes et/ou de sucre.

**2** - Procédé selon la revendication 1 caractérisé en ce qu'on pulvérise sur des granulés de lysine et/ou de méthionine une solution de chlorhydrate de lysine.

**3** - Procédé selon la revendication 1 caractérisé en ce que l'on pulvérise sur des cristaux de chlorhydrate de lysine une solution aqueuse de chlorhydrate de lysine.

**4** - Procédé selon l'une quelconque des revendications 1 à 3 caractérisé en ce qu'on pulvérise sur des granulés de lysine et/ou de méthionine une solution aqueuse contenant entre 70 et 150 g de chlorhydrate de lysine pour 100 g d'eau.

**5** - Procédé selon la revendication 4 caractérisé en ce qu'on pulvérise sur des granulés de lysine et/ou de méthionine une solution selon la revendication 4 maintenue à une température comprise entre 50 et 70°C quand la concentration en chlorhydrate de lysine est supérieure à 100 g pour 100 ml d'eau.

**6** - procédé selon l'une quelconque des revendications précédentes caractérisé en ce qu'on pulvérise sur des granulés de lysine et/ou de méthionine une solution selon la revendication 4 ou 5 en quantité telle que la quantité de chlorhydrate de lysine pulvérisée exprimée en matière sèche par rapport au granulé de départ est comprise entre 10 et 150 % et de préférence entre 25 et 110 %.

**7** - Procédé selon les revendications 3 et 6 caractérisé en ce que la quantité pondérale de chlorhydrate de lysine pulvérisée par rapport au granulé de départ est comprise entre 70 et 100 %.

**8** - Granulés de principes actifs caractérisés en ce qu'ils contiennent un coeur de principes actifs lissés par une couche de lysine déposée par le procédé de l'une quelconque des revendications 1 à 7.

**9** - Granulés selon la revendication 8 caractérisés en ce qu'ils sont constitués de 50 à 90 % de principes actifs en poids et de 10 % à 50 % de chlorhydrate de lysine.

**10** - Granulés selon l'une quelconque des revendications précédentes caractérisés en ce qu'ils présentent une granulométrie comprise entre 0,5 et 2,5 mm.

**11** - Utilisation des granulés selon l'une des revendications 8 à 10 pour l'enrobage par un ou plusieurs polymères ou un copolymère ayant des groupes amino basiques en mélange éventuellement avec une substance hydrophobe.

**12** - Utilisation des granulés selon la revendication 11 pour l'enrobage par un mélange de copolymère à base de vinylpyridine et de styrène et d'acide stéarique.

**13** - Granulés obtenus selon la revendication 12 caractérisés en ce que la quantité pondérale d'enrobant par rapport aux granulés des revendications 8 à 10 est comprise entre 10 et 30 %.

**14** - Granulés obtenus selon les revendications 11 à 13 caractérisés en ce que la couche d'enrobage a une épaisseur comprise entre 20 et 70 µm.

Office européen
des brevets

**RAPPORT DE RECHERCHE EUROPEENNE**

Numero de la demande

EP 91 40 1798

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 094 123 (THE PROCTER & GAMBLE CO.) <br> * Tout le document, en particulier page 5, lignes 14-18 * <br> --- | 1 | A 61 K 9/16 <br> A 61 K 9/50 <br> A 61 K 9/52 <br> A 61 K 9/54 <br> A 23 K 1/00 |
| X | EP-A-0 351 760 (MITSUBISHI KASEI CORP.) <br> * Page 2, lignes 1-4; page 2, ligne 42 - page 3, ligne 12; page 3, ligne 56 - page 4, ligne 20; pages 7,8, exemple 1; revendications 1-4,10-13 * | 1 | |
| Y | | 2,8-14 | |
| Y | GB-A-1 134 793 (KYOWA HAKKO KOGYO K.K.) <br> * Page 1, ligne 41- page 2, ligne 9; page 2, exemple 1; revendications 1,7,9,11,18 * <br> --- | 2,8-14 | |
| A | EP-A-0 260 186 (RHONE-POULENC SANTE) <br> --- | | |
| A | DE-A-3 532 692 (BOEHRINGER MANNHEIM GmbH) <br> * Page 3, lignes 40-42 * <br> ----- | 1 | **DOMAINES TECHNIQUES RECHERCHES (Int. Cl.5)** <br><br> A 61 K <br> A 23 K |

Le présent rapport a été établi pour toutes les revendications

| Lieu de la recherche | Date d'achèvement de la recherche | Examinateur |
|---|---|---|
| LA HAYE | 24-09-1991 | BOULOIS D.J-M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)